# EUROPEAN PATENT SPECIFICATION

(11) **EP 1 274 351 B1**
(45) Date of publication and mention of the grant of the patent: **10.09.2008**
(21) Application number: 01969028.8
(22) Date of filing: 18.04.2001
(51) Int. Cl.: A61B 17/04

(54) **APPARATUS FOR SUTURING**
VORRICHTUNG ZUM NÄHEN VON GEWEBE
APPAREIL DE SUTURE

(30) Priority: 18.04.2000 US 550975
(43) Date of publication of application: 15.01.2003
(60) Divisional application: 08000459.1
(73) Proprietor: Orthopaedic Biosystems Limited, Inc., Memphis, TN 38116 (US)
(72) Inventor: SKIBA, Jeffrey, B., Oracle, AZ 85623 (US); BALDWIN, Jeffrey, P., Phoenix, AZ 85027 (US); HATCH, Laird, L., Cave Creek, AZ 85331 (US); GARTSMAN, Gary, M., Houston, TX 77030 (US); OREN, Ran, 25130 Gaaton (IL)
(74) Representative: Connors, Martin L.H.
(86) International application number: PCT/US2001/040541
(87) International publication number: WO 2001/078609

(56) References cited:
- WO-A-92/05828
- WO-A-95/11630
- WO-A-96/39946
- WO-A-97/16121
- DE-A- 2 532 242
- DE-U- 29 722 769
- FR-A- 401 199
- GB-A- 528 550
- US-A- 2 737 954
- US-A- 5 149 329
- US-A- 5 222 977
- US-A- 5 391 174
- US-A- 5 522 820
- US-A- 5 562 685
- US-A- 5 843 099

## Description

The present invention relates to an apparatus for use in surgical suturing.

Suture is often used to bring two pieces of tissue together and hold the tissues as they heal. Needles allow a surgeon to pierce tissue and pull suture through that tissue. In many procedures, there is adequate space in the wound or incision to manipulate needles and needle holding instruments. The move by surgeons toward less invasive and minimally invasive surgeries has created the need for instruments to be designed to function in limited space and with longer reaches. Standard surgical instruments are being stretched and downsized to pass through tunnels in the tissue called cannulas or portals measuring as small as 5 millimetres in diameter and in a variety of lengths. There is a need for improved surgical instruments to manipulate and control sutures during surgical operations.

FR401,199 describes surgical needles that can trap sutures using a notch mechanism near the tip of the needle.

WO95/11630 describes a suture needle with a notch mechanism near the tip of the needle that is optionally combined with a lath to hold the suture in place.

US 5,562,685 describes a suture needle with an opening near the tip of the needle.

### SUMMARY OF THE INVENTION

The present invention provides new and useful devices for use in suturing. These devices are particularly useful in laparoscopic, arthroscopic, and/or open surgical procedures.

The invention is as claimed in claim 1.

One type of suturing device according to the invention is characterized by a handle with an elongated shaft with a sharpened tip configured to hold a suture at a selected point on the suture. Several different types of structures for holding the suture to the sharp tip are provided according to the principles of the invention. For example, one such structure comprises a tapered opening formed in the tip, and configured to enable a suture to be wedged and held in the opening. In another example, an elongated opening is provided, which may be curved, for holding the suture. In yet another example, a locking mechanism is provided, which has a locked position for holding the suture, an unlocked position for releasing the suture, and a partially locked position for controlled adjustment of the suture position relative to the tip.

Another type of device according to the principles of the invention is characterized by new and useful structures and techniques for loading the suture into the device. In one example of this aspect of the present invention, a suture may be side-loaded into the device. This provides numerous advantages, including eliminating the need to thread the suture, and allowing side-release of the suture.

According to the present invention, different shaped suture tips are provided for the selection of the most effective tip shape for a given application. In one example of the present invention, each tip may be detached from the suturing device and replaced by another tip. In another example of the present invention, the position of each tip is adjustable relative to the shaft of the suturing device for further refinement of the suturing device for a given application.

A suturing device according to the present invention may also be configured for use with a needle point swedged to a suture.

Yet another type of suturing device includes a tissue support structure disposed adjacent the sharpened tip, which may move relative to the sharpened tip to facilitate penetration of the tip through a tissue. In one example of this, a suture is carried through the tissue by the tip as the tip penetrates the tissue. In another example of this, the tip engages the suture after penetration of the tissue, and draws the suture through the tissue as the tip is withdrawn from the tissue.

These and other features of the present invention will become further apparent from the following detailed description and the accompanying drawings.

### BRIEF DESCRIPTION OF THE DRAWINGS

A more complete understanding of the present invention may be derived by referring to the detailed description and claims when considered in connection with the following illustrative Figures, which may not be to scale. In the following Figures, like reference numbers refer to similar elements throughout the Figures.
FIG. 1 is a perspective view of a suturing device;
FIG. 2 is a close-up perspective view of the sharpened tip at the distal end of the suturing device of FIG. 1;
FIGS. 3-5 illustrate the relative positions of a suture and a suturing device during coupling of a suture with the suturing device;
FIGS. 6A-6B schematically illustrate the manner in which a suture is trapped and held by an opening in the sharpened tip of a suturing device;
FIGS. 7-10 illustrate successive steps in applying a suture to tissue using a suturing device;
FIGS. 11-12 schematically illustrate sharpened tips and different configurations for an opening for use in coupling a suture with the sharpened tip (Fig.12 in accordance with the principles of the present invention);
FIGS. 13-17 schematically illustrate sharpened tips and different configurations for an opening for use in coupling a suture with the sharpened tip;
FIGS. 18-21 schematically illustrate a suturing device with a locking mechanism;
FIG. 22 schematically illustrates a sharpened tip with a combination of openings for use in coupling a suture;
FIGS. 23-29 schematically illustrate different configurations for a sharpened tip with an opening for coupling a suture with the sharpened tip;
FIGS. 30-32 schematically illustrate different ways for a coupling a needle with a swedged suture to a suturing device;
FIG. 33 schematically illustrates another way for coupling a suture to a suturing device; and
FIG. 34-36 illustrate a suture device in which a tissue support device is provided.

As set forth above, the present invention provides several types of devices and techniques which are useful in suturing, for example in connection with laparoscopic, arthroscopic, and/or open surgical procedures. Examples of devices according to the principles of the present invention are described below in connection with particular embodiments, but it will be clear to those skilled in the art that aspects of the present invention found in one example may be practiced alone or in combination with (or even in lieu of) devices.

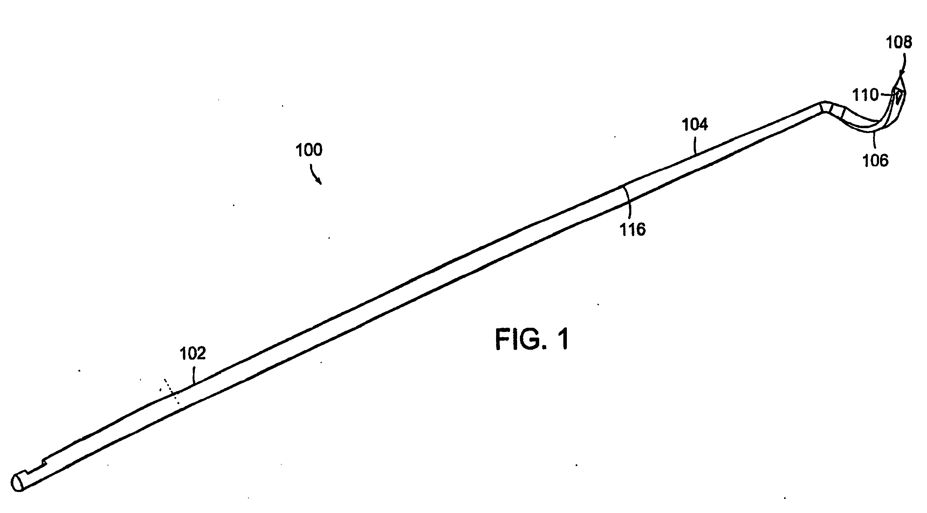

One type of suturing device is shown in FIG.1. Suturing device 100 has a handle 102, a shaft 104 connected to the handle, and a tip 106 connected to the other (distal) end of the shaft. Typically, the handle 102 may be 10.16 to 15.24 cm (4 to 6 inches) long, and the shaft 104 may typically be from 2.54 to 60.96 cm (1 to 24 inches), depending on the application. The shaft is typically 2 to 3 millimetres in diameter and formed of a rigid substance. Tip 106 includes a sharpened point 108 and an eyelet (or opening) 110.

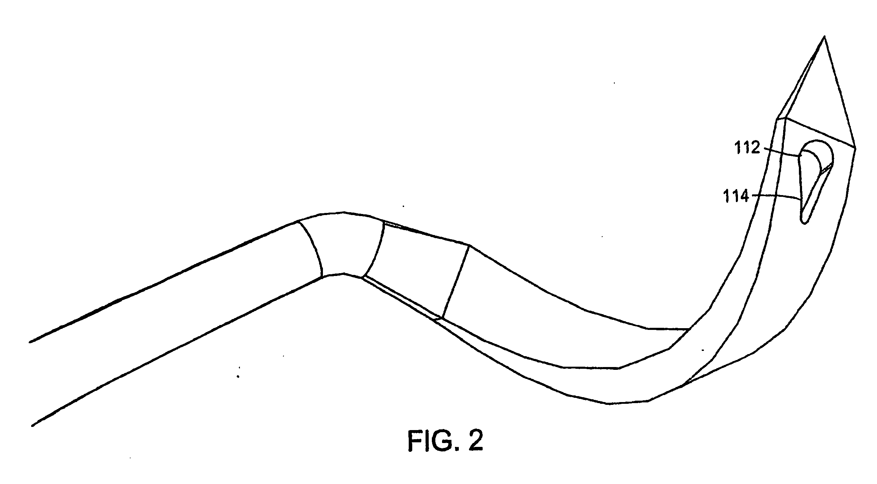

FIG. 2 shows a close-up view of tip 106. Tip 106 has a curved, hook-like shape for convenient delivery of a suture, as discussed further below. Eyelet 110 has a top portion 112 configured to be wider than a suture used in connection with the suturing device, and a narrowed bottom portion 114 for trapping the suture.

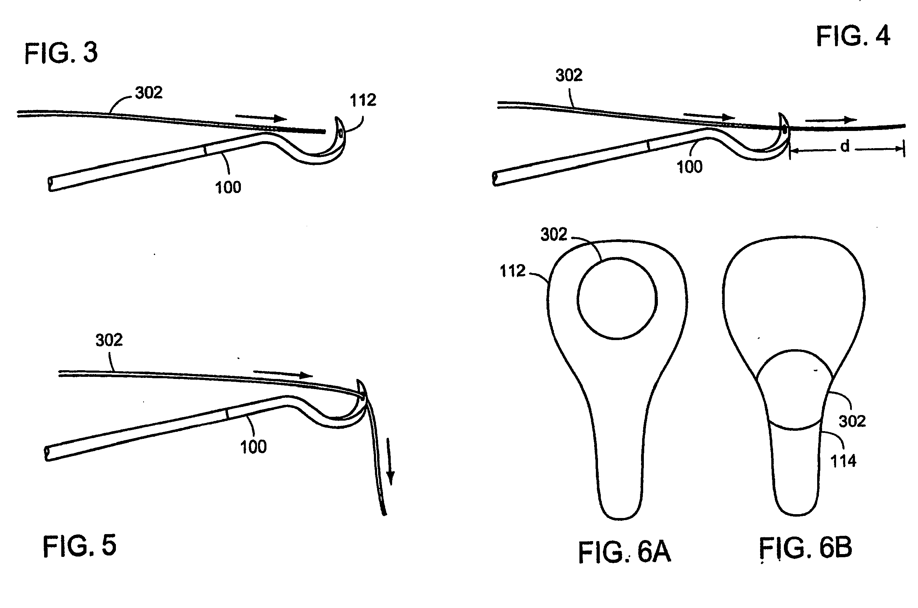

Referring now to FIGS. 3 through 6B, a method is shown. Suture 302 is threaded through eyelet 110 as shown in FIG. 3 and 4 until a selected length of suture (labelled "d") is extended through suturing device 100 as shown in FIG. 4. Preferably, length d is about 5 cm (2 inches). Once the selected point of suture is in eyelet 110, length d is drawn downward, as shown in FIG. 5, trapping the suture in eyelet 110. FIG 6A shows the position of the suture in the wider top portion 112 of eyelet 110 prior to the drawing downward step. FIG. 6B shows the suture wedged into the narrowed bottom portion 114 of eyelet 110 after the downward drawing step of FIG 5. The suture in FIG. 6B is thus trapped in the narrowed bottom portion 114.

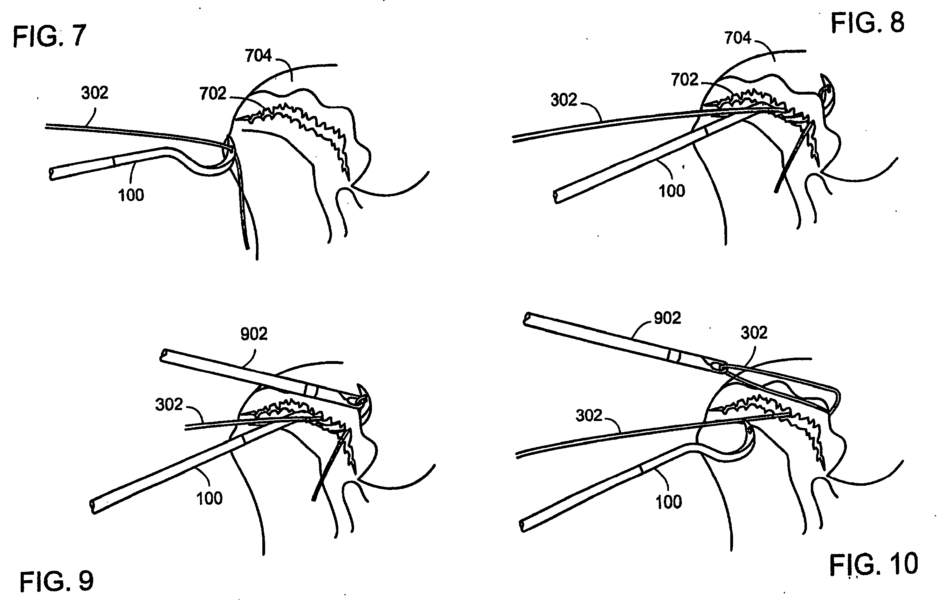

Figs. 7 through 10 show in the repair of a gap 702 formed in tissue 704. As shown in FIGS. 7 and 8, suturing device 100 with suture 302 is partially inserted into a portion of tissue 704. Sharpened tip 106 of suturing device 100 penetrates tissue 704, thereby delivering suture 302 to the other side of tissue 704. A second tool 902 (FIG. 9) is used to assist in the release of suture 302 from eyelet 110, and to draw the short end of suture 302 the rest of the way through tissue 704 and fully disengage suture 302 from device 100 (see Fig. 10).

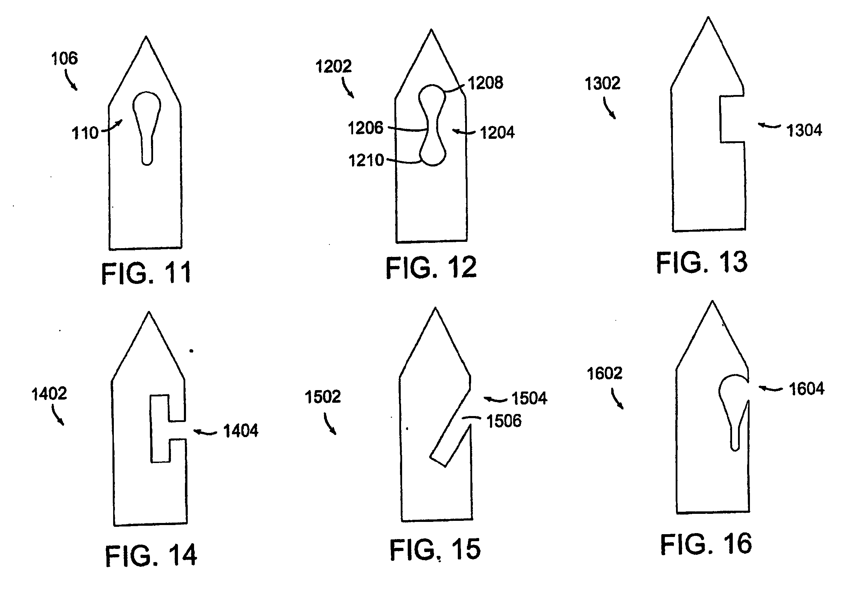

FIG. 11 shows a close-up of a tapered eyelet 110 of the type described in FIGS. 1 through 10, and further illustrating the orientation of the tapered eyelet 110 relative to the sharpened tip 106.

FIG. 12 shows a close-up of a sharpened tip 1202 with an alternative embodiment of an eyelet 1204 in accordance with the principles of the present invention. Eyelet 1202 has a tapered central portion 1206 which may trap a suture from either the top wider portion 1208 or the bottom wider portion 1210 of eyelet 1204. One advantage of this embodiment is that the device operator may disengage suture 302 from device 100 either by pulling the suture towards the wider top portion 1208 or the wider bottom portion 1210. It will be clear to one skilled in the art that this embodiment of the present invention provides added flexibility in the manner in which the suture can be released.

FIG. 13 shows a close-up of a sharpened tip 1302 with an eyelet 1304. Eyelet 1304 is configured for side-loading of the suture into the eyelet. Eyelet 1304 is advantageous where the end of a suture is frayed, or it is otherwise difficult or time-consuming to pass lengthwise through an eyelet (as shown in FIGS. 3 and 4). The eyelet configuration shown in FIG. 13 allows for very easy loading, sliding, and release of the suture from the suturing device.

FIG. 14 shows another sharpened tip 1402 with an eyelet 1404 with the feature of side-loading of a suture. The T-shaped configuration of eyelet 1404 substantially traps the suture in eyelet 1404 while allowing the suture to slide lengthwise through the eyelet.

FIG. 15 shows yet another sharpened tip 1502 with another configuration of an eyelet 1504 with side-loading capability. Eyelet 1504 is configured with a deep diagonal slot 1506 which substantially traps the suture while allowing the suture to slide lengthwise through the eyelet.

FIG. 16 shows yet another sharpened tip 1602 with an eyelet 1604 with side-loading capability. In FIG. 16, eyelet 1604 has a tapered configuration so that a suture may be side-loaded into a suturing device and then wedged into the tapered portion of the eyelet for improved trapping of the suture.

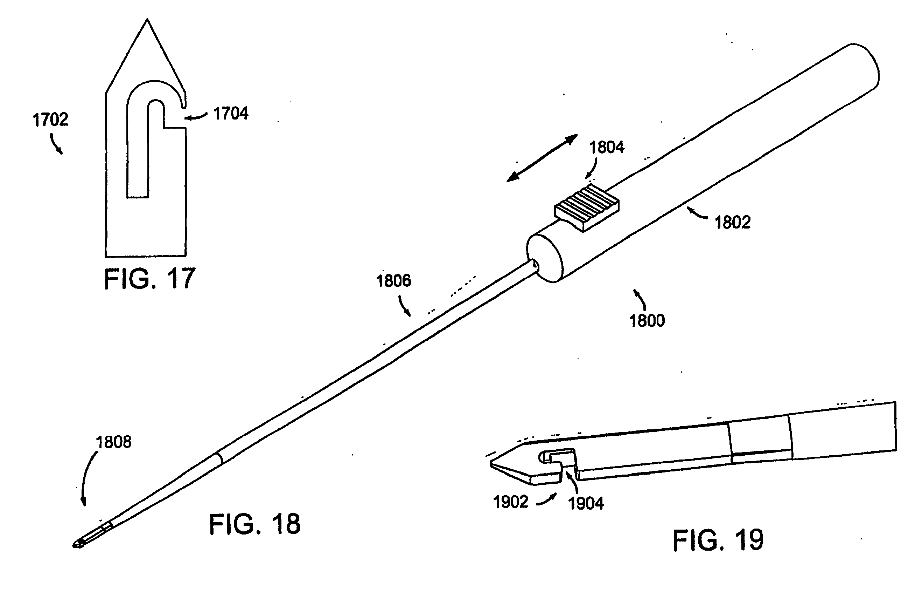

FIG. 17 shows yet another sharpened tip 1702 with an eyelet 1704 with a side-loading capability. In FIG. 17, eyelet 1704 has an elongated and curved configuration for substantially trapping a suture while allowing the suture to slide lengthwise through the eyelet. The elongated and curved configuration of the eyelet makes it very unlikely that a suture will be unintentionally released from the suturing device.

Figures 18 through 21 illustrate a suturing device with a locking mechanism for trapping a suture in an opening in a sharpened tip. As shown in FIG. 18, suturing device 1800 has a handle 1802 with locking mechanism button 1804, shaft 1806, and sharpened tip 1808.

FIG. 19 illustrates a close-up of sharpened tip 1808, having opening 1902 and locking element 1904. Locking element 1904 is controlled by the device operator with button 1804 (FIG. 18). Locking element 1804 may be selectively placed in (1) an open position for inserting a suture into an opening in the sharpened tip and for release of the suture from the opening in the sharpened tip; (2) at least one partially closed position for capturing the suture and for controlled adjustment of the suture relative to the tip; and (3) a locked position for trapping a suture in the opening in the sharpened tip at a selected point.

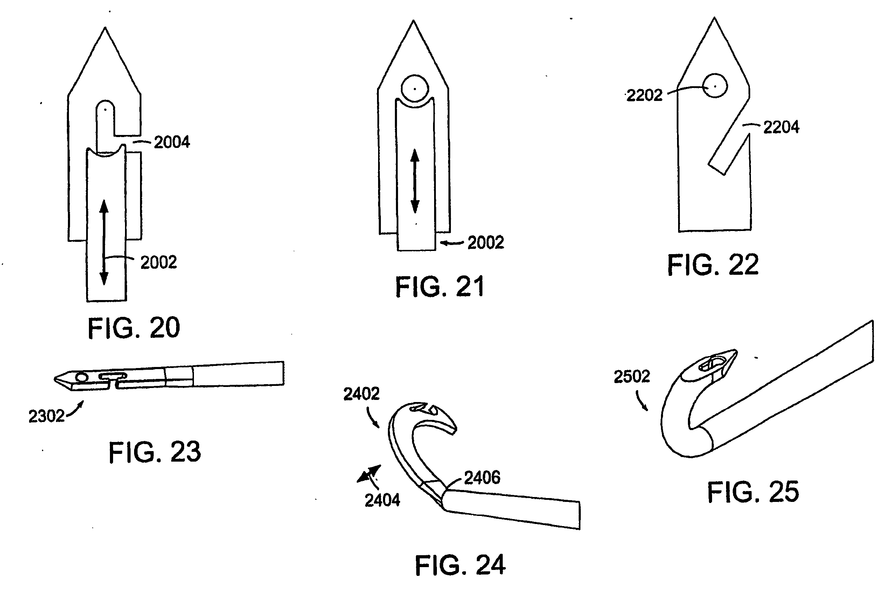

FIG. 20 further illustrates the use of the locking mechanism. FIG. 20 shows a schematic view if the sharpened tip 1808 with opening 1902. Sharpened tip 1808 has side-loading capability and locking element 1904, which can be selectively placed in any of the foregoing positions by movement of the locking element in the direction of arrow 2002. When a suture s first side-loaded, locking element 1904 is in the open position as shown in FIG. 20. As locking element 1904 is pushed forward and the side-opening 2004 is blocked-off, the suture is captured in the tip, but is not yet trapped in a particular position. In this state, the suture may slide lengthwise relative to sharpened tip 1808, but may not be removed from sharpened tip 1808 unless the full length of the suture is drawn through opening 1902. As element 1904 is moved further forward, it engages the suture with a light amount of friction, giving the device operator the ability to control the lengthwise movement of the suture without damaging the suture. Finally as locking element 1904 is pushed fully forward, the suture becomes wedged in opening 1902, and fixed in a locked position. Release of the suture is achieved by moving the locking button to move the locking element away from the locked position.

In the suturing device shown in FIG. 21, the locking element moves in the same manner as in FIG. 20, and the same locking principles apply, but no side-loading capability is provided.

Referring to FIG. 22, various combinations of eyelets may be formed on a sharpened tip. For example, a round eyelet 2202 may be formed in addition to a deep diagonal slot 2204 on a device tip to allow selection of fully enclosing a suture (round eyelet) or side-loading of a suture with substantial trapping of the suture (deep diagonal slot) on the same tool. As another example, the eyelet with a tapered central portion as shown in FIG. 12 may also be combined with a side-loading feature.

Figures 23 through 29 show different sharpened tips for use in different applications. The sharpened tip 2302 illustrated in FIG. 23 extends substantially straight and forward from the distal end of the device shaft. Alternatively, as shown in FIG. 24, as the sharpened tip 2402 may be angularly bent relative to the shaft in a selected direction, and angled to a side. As illustrated in FIG. 25, and sharpened tip 2502 may be further bent so that the distal portion of the tip generally points towards the proximal end of the shaft.

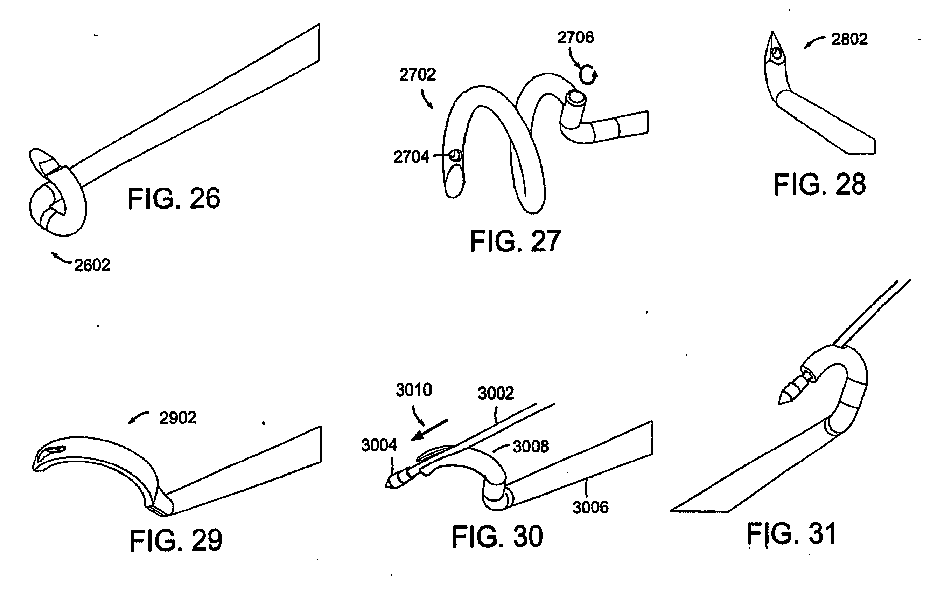

Referring now to FIG. 26, an alternative is shown wherein a sharpened tip 2602 is curved at least partially about the distal end of the shaft.

In to FIG. 27, a sharpened tip 2702 is formed with at least one spiral loop at the distal end of the shaft. In the illustrated example, the spiral shaped tip 2702 has about 1.5 loops. With a suture held near the front of the spiral (see location of eyelet 2704), the suture may be passed through a tissue two or more times prior to releasing the stitch from a suturing device.

As shown in FIG. 28, a sharpened tip 28 may be configured to extend directly to one side of distal end of the shaft.

Further, as shown in FIG.29, a sharpened tip 2902 may be configured to extend at least partially forward from the distal end of the shaft with a concave configuration.

Each of the above sharpened tip configurations may be selected for a particular application to allow a surgeon to pierce tissue in precisely the position chosen for the optimal re-approximation of tissue. Further, each of the above sharpened tip configurations may be combined with the other various aspects of the present invention to provide a wide selection of combinations to best pass suture through a substrate. For example, each of the above sharpened tip configurations may be combined with any of the eyelet configurations. Moreover, any of the eyelets could have side-loading capability and/or a locking mechanism.

In accordance with a further principle of the present invention, each tip may be configured for adjustability relative to the axis of the suturing device shaft, for providing further flexibility as to the orientation in which the suture may be applied to a gap in tissue. For example, the angle of the tip in FIG. 24 relative to the shaft may be adjusted at point in the direction of arrow 2404. Similarly, the looped tip in FIG. 27 may be rotated in the direction of arrow 2706. Each of the sharpened tips disclosed thus may be pivoted, rotated, or otherwise further adapted for improved application of the sharpened tip and the suture.

In accordance with a further principle of the present invention, various tips may be removed from the suturing device and replaced with an alternative tip or with a new, sharper tip. For example, referring to FIG. 24, the tip may be removed at point 2406 and replaced with the tip as shown in FIG. 25. Alternatively, the tip may be detached at other positions on the suturing device, such as by removing the shaft at point 116 (FIG. 1), and replacing it with a shaft carrying a differently shaped tip.

Referring now to FIGS. 30 through 32, further embodiments configured for use with a needle point swedged to a suture are shown. As shown in FIG. 30, suture 3002 with needle tip 3004 may be side-loaded into a suturing device with a shaft 3006 with a curved distal end portion 3008. In accordance with this embodiment, needle tip 3004 is sharpened and acts to penetrate and lead suture 3002 through a tissue. Once the tissue is punctured, the needle tip is grasped with a suture grasper (e.g. similar to second tool 902 in FIG. 9) and removed from the shaft in the direction of arrow 3010. The shaft is withdrawn and the suture is pulled through the tissue to complete the stitch. This embodiment also allows the simple replacement of sharp needles for tips that may have become dull. Further, tissue abrasion associated with pulling the short end of a suture through an opening already containing a length of suture is eliminated.

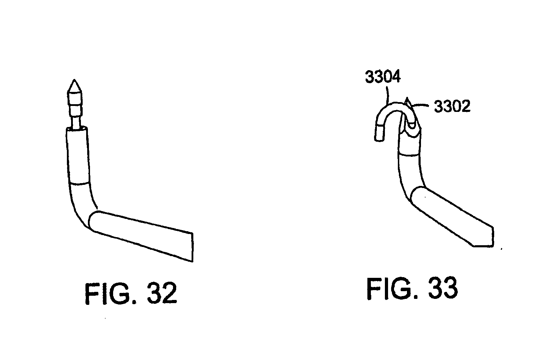

The shaft as in FIG. 30 may be modified as a cannula with the suture passing through a partial length of the device (FIG. 31) or the full length of the device (FIG. 32). These structures are advantageous in reducing or eliminating the amount of loose suture from the procedure area. Each of the devices in FIGS. 30 through 32 will preferably have a configuration for firmly holding the needle tip while it is piercing the tissue.

Referring to FIG. 33, alternatively, a suturing device 3300 may have a sharpened tip 3302, as in FIGS. 1 through 29, and be configured as a cannula with the suture passing through the full length of the device with a section 3304 protruding from the tip, in position for grasping as soon as the sharpened tip pierces a substrate. Section 3304 is located behind the first portion of the tip sharpened tip that pierces a tissue, so that the outwardly extending portion is carried through the tissue which has been pierced by the sharpened tip.

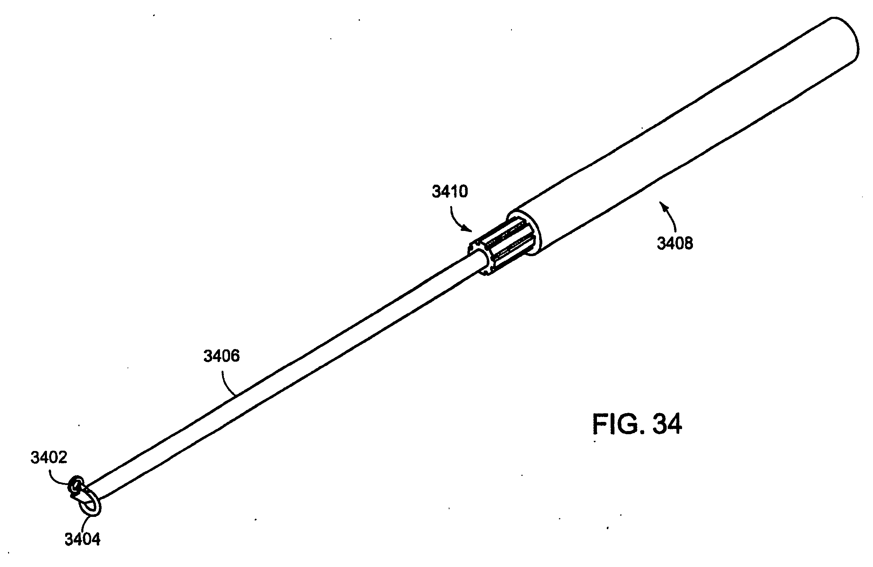

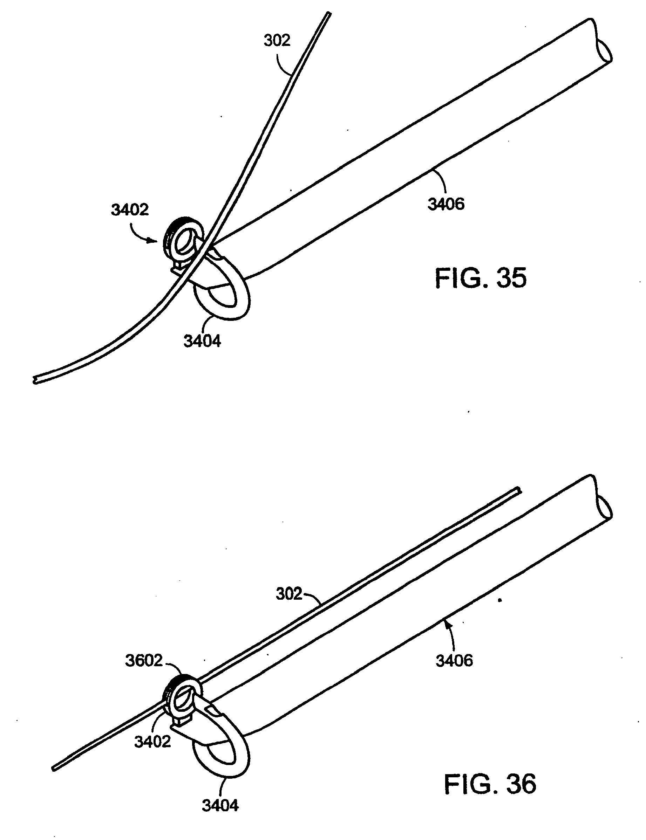

Referring now to FIG. 34, another suturing device including a tissue support structure 3402 disposed adjacent sharpened tip 3404 is shown. Tissue support structure 3402 is located on rotatable sheath 3406, which encases a shaft (shaft is not shown but is attached to handle 3408 and tip 3404). Rotatable sheath 3406 is preferably rotated by turning serrated handle 3410. Referring briefly to FIG. 35, a close-up view of the distal tip of a suturing device according to the principles of FIG. 34 is shown.

On occasion, the tissue to be sutured is difficult to pierce and requires pressure from behind to "push" the tissue against the sharpened tip. Use of the tissue support structure 3402 is accomplished by positioning a portion of tissue to be sutured in between sharpened tip 3404 and tissue support 3402. The sharpened tip is rotated through the tissue while the tissue supports rotates in an opposite direction, forcing the tissue against and to be pierced by the sharpened tip. The relative rotational motion between tissue support structure 3402 and sharpened tip 3404 provides the device operator with tactile feedback as to the amount of force being applied to a tissue.

Tissue support structure 3402 and sharpened tip 3404 are suitably configured to approach and engage a portion of tissue from a broad range of angles or positions. Combination the various sharpened tip configurations in accordance with preferred embodiments of the present invention an the adjustability of those tips with the tissue support structure provides an even broader range of options for engaging a tissue between a sharpened tip and tissue support structure 3402.

With continue reference to FIG. 35, a suture 302 is connected with the sharpened tip and passed through the tissue as the sharpened tip pierces the tissue. After piercing, the suture is grasped on the other side of the tissue, as described above, released from the sharpened tip, and then pulled through the tissue for further use in suturing.

Referring now to FIG. 36, an alternative configuration for drawing a suture through a tissue is disclosed. A suture 302 is connected with a sling 3602 in a tissue support device 3604. The sharpened tip pierces the tissue with the assistance of the tissue support device, and engages the suture as the sharpened tip is drawn back through the tissue and the suturing device is rotated open. In this way, if a suture is preferably applied from a side of a tissue inaccessible to the sharpened tip, the sharpened tip may be engaged from the opposite (and accessible) side of the tissue, and then made to draw the suture back through the tissue.

The present invention has been described above with reference to preferred embodiments. However, those skilled in the art having read this disclosure will recognize that changes and modifications may be made to the preferred embodiment without departing from the scope of the present invention. For example, the combination of the various opening configurations with the configurations of the sharpened tip is clearly contemplated by this disclosure. Similarly, the use of the various eyelets, tip configurations, and locking mechanisms with the different tissue support features is intended. These and other changes or modifications are intended to be included within the scope of the present invention, as expressed in the following claims.

## Claims

1. A suturing device (100) comprising:
a handle (102);
an elongated shaft (104) attached to said handle (102); and
a sharpened tip (106) on said elongated shaft (104), wherein said sharpened tip (106) includes an eyelet (110) to allow enclosing of a suture (302) **characterised in that** the eyelet (110) has a tapered central portion (1206), a first wider portion (1208) and a second wider portion (1210) wherein the central portion may trap a suture (302) from either the first or second portion, and wherein the suture (302) is disengaged from the device by pulling the suture (302) towards either the first or second wider portions.

2. A suturing device as claimed in claim 1, wherein said tapered central portion (1206) may trap a suture (302) from either a top wider portion (1208) or a bottom wider portion (1210) of the eyelet (1204).

3. A suturing device as claimed in any preceding claim, wherein the eyelet may also be combined with a side loading feature.

4. A suturing device as claimed in any preceding claim, wherein said sharpened tip has a hooked configuration.

5. A suturing device as claimed in any preceding claim, wherein said sharpened tip is angularly bent relative to said shaft in a selected direction.

6. A suturing device as claimed in any preceding claim, wherein said sharpened tip is configured with at least one spiral loop at the distal end of the shaft.

7. A suturing device as claimed in any preceding claim, wherein said sharpened tip is bent so the distal tip generally points towards the proximal end of the shaft.

8. A suturing device as claimed in any preceding claim, wherein said sharpened tip is curved at least partially about the distal end of the shaft.

9. A suturing device as claimed in any preceding claim, wherein said sharpened tip extends at an angle and to one side of the distal end.

10. A suturing device as claimed in any preceding claim, wherein said sharpened tip extends at least partially forward from the distal end with a concave configuration.

11. A suturing device as claimed in any preceding claim, wherein said sharpened tip extends substantially straight and forward from said distal end.

12. A suturing device as claimed in any preceding claim, wherein said sharpened tip is selectively moveable relative to said shaft.

13. A suturing device as claimed in any preceding claim, wherein said sharpened tip is selectively detachable from said shaft.

14. A suturing device as claimed in any preceding claim, wherein the suture (302) comprises a length of material, and wherein said eyelet (110) is configured to allow the suture to pass lengthwise through said eyelet (110).

## Patentansprüche

1. Eine Nähvorrichtung (100), die Folgendes beinhaltet:
einen Griff (102);
einen verlängerten Stiel (104), der an dem Griff (102) befestigt ist; und
eine zugespitzte Spitze (106) auf dem verlängerten Stiel (104), wobei die zugespitzte Spitze (106) eine Öse (110) umfasst, um das Umschließen eines Nahtmaterials (302) zu ermöglichen, **dadurch gekennzeichnet, dass** die Öse (110) einen verjüngten zentralen Abschnitt (1206), einen ersten breiteren Abschnitt (1208) und einen zweiten breiteren Abschnitt (1210) aufweist, wobei der zentrale Abschnitt ein Nahtmaterial (302) von entweder dem ersten oder dem zweiten Abschnitt abfangen kann, und wobei das Nahtmaterial (302) durch das Ziehen des Nahtmaterials (302) in Richtung des entweder ersten oder zweiten breiteren Abschnitts von der Vorrichtung gelöst wird.

2. Nähvorrichtung gemäß Anspruch 1, wobei der verjüngte zentrale Abschnitt (1206) ein Nahtmaterial (302) von entweder einem oberen breiteren Abschnitt (1208) oder einem unteren breiteren Abschnitt (1210) der Öse (1204) abfangen kann.

3. Nähvorrichtung gemäß einem der vorhergehenden Ansprüche, wobei die Öse ebenfalls mit einem Merkmal zur seitlichen Zuführung kombiniert werden kann.

4. Nähvorrichtung gemäß einem der vorhergehenden Ansprüche, wobei die zugespitzte Spitze eine hakenförmige Konfiguration aufweist.

5. Nähvorrichtung gemäß einem der vorhergehenden Ansprüche, wobei die zugespitzte Spitze relativ zu dem Stiel in einer ausgewählten Richtung winklig gebogen ist.

6. Nähvorrichtung gemäß einem der vorhergehenden Ansprüche, wobei die zugespitzte Spitze an dem distalen Ende des Stiels mit mindestens einer spiralförmigen Schleife konfiguriert ist.

7. Nähvorrichtung gemäß einem der vorhergehenden Ansprüche, wobei die zugespitzte Spitze gebogen ist, so dass die distale Spitze im Allgemeinen in Richtung des proximalen Endes des Stiels deutet.

8. Nähvorrichtung gemäß einem der vorhergehenden Ansprüche, wobei die zugespitzte Spitze zumindest teilweise um das distale Ende des Stiels gekrümmt ist.

9. Nähvorrichtung gemäß einem der vorhergehenden Ansprüche, wobei sich die zugespitzte Spitze in einem Winkel und zu einer Seite des distalen Endes erstreckt.

10. Nähvorrichtung gemäß einem der vorhergehenden Ansprüche, wobei sich die zugespitzte Spitze mit einer konkaven Konfiguration zumindest teilweise von dem distalen Ende nach vorne erstreckt.

11. Nähvorrichtung gemäß einem der vorhergehenden Ansprüche, wobei sich die zugespitzte Spitze von dem distalen Ende im Wesentlichen gerade und nach vorne erstreckt.

12. Nähvorrichtung gemäß einem der vorhergehenden Ansprüche, wobei die zugespitzte Spitze relativ zu dem Stiel selektiv beweglich ist.

13. Nähvorrichtung gemäß einem der vorhergehenden Ansprüche, wobei die zugespitzte Spitze von dem Stiel selektiv abnehmbar ist.

14. Nähvorrichtung gemäß einem der vorhergehenden Ansprüche, wobei das Nahtmaterial (302) eine Länge an Material beinhaltet, und wobei die Öse (110) konfiguriert ist, um dem Nahtmaterial zu ermöglichen, der Länge nach durch die Öse (110) geführt zu werden.

## Revendications

1. Un dispositif de suture (100) comprenant :
une poignée (102) ;
un arbre allongé (104) attaché à ladite poignée (102) ; et
un bout taillé en pointe (106) sur ledit arbre allongé (104), dans lequel ledit bout taillé en pointe (106) inclut un oeillet (110) afin de permettre d'enclore un fil de suture (302) **caractérisé en ce que** l'oeillet (110) a une portion centrale effilée (1206), une première portion plus large (1208) et une deuxième portion plus large (1210), dans lequel la portion centrale peut retenir un fil de suture (302) et l'empêcher d'aller soit dans la première, soit dans la deuxième portion, et dans lequel le fil de suture (302) est dégagé du dispositif en tirant le fil de suture (302) en direction soit de la première, soit de la deuxième portion plus large.

2. Un dispositif de suture tel que revendiqué dans la revendication 1, dans lequel ladite portion centrale effilée (1206) peut retenir un fil de suture (302) et l'empêcher d'aller soit dans une portion plus large de dessus (1208), soit dans une portion plus large de dessous (1210) de l'oeillet (1204).

3. Un dispositif de suture tel que revendiqué dans n'importe quelle revendication précédente, dans lequel l'oeillet peut également être combiné à une caractéristique de chargement latéral.

4. Un dispositif de suture tel que revendiqué dans n'importe quelle revendication précédente, dans lequel ledit bout taillé en pointe est configuré en crochet.

5. Un dispositif de suture tel que revendiqué dans n'importe quelle revendication précédente, dans lequel ledit bout taillé en pointe est coudé de façon angulaire par rapport audit arbre dans une direction sélectionnée.

6. Un dispositif de suture tel que revendiqué dans n'importe quelle revendication précédente, dans lequel ledit bout taillé en pointe est configuré avec au moins une boucle en spirale au niveau de l'extrémité distale de l'arbre.

7. Un dispositif de suture tel que revendiqué dans n'importe quelle revendication précédente, dans lequel ledit bout taillé en pointe est coudé de sorte que le bout distal pointe d'une manière générale en direction de l'extrémité proximale de l'arbre.

8. Un dispositif de suture tel que revendiqué dans n'importe quelle revendication précédente, dans lequel ledit bout taillé en pointe est courbé au moins partiellement autour de l'extrémité distale de l'arbre.

9. Un dispositif de suture tel que revendiqué dans n'importe quelle revendication précédente, dans lequel ledit bout taillé en pointe s'étend à un angle et sur un côté de l'extrémité distale.

10. Un dispositif de suture tel que revendiqué dans n'importe quelle revendication précédente, dans lequel ledit bout taillé en pointe s'étend au moins partiellement vers l'avant depuis l'extrémité distale avec une configuration concave.

11. Un dispositif de suture tel que revendiqué dans n'importe quelle revendication précédente, dans lequel ledit bout taillé en pointe s'étend de façon substantiellement droite et vers l'avant depuis ladite extrémité distale.

12. Un dispositif de suture tel que revendiqué dans n'importe quelle revendication précédente, dans lequel ledit bout taillé en pointe peut être déplacé de façon sélective par rapport audit arbre.

13. Un dispositif de suture tel que revendiqué dans n'importe quelle revendication précédente, dans lequel ledit bout taillé en pointe peut être détaché de façon sélective dudit arbre.

14. Un dispositif de suture tel que revendiqué dans n'importe quelle revendication précédente, dans lequel le fil de suture (302) comprend une longueur de matériau, et dans lequel ledit oeillet (110) est configuré pour permettre au fil de suture de passer dans le sens de la longueur au travers dudit oeillet (110).
